# EUROPEAN PATENT SPECIFICATION

(11) **EP 3 116 473 B1**
(45) Date of publication and mention of the grant of the patent: **30.12.2020**
(21) Application number: 15712008.0
(22) Date of filing: 11.03.2015
(51) Int. Cl.: A61K 9/10, A61K 9/00, A61K 47/10, A61K 47/20, A61K 31/57, A61K 31/573, A61P 17/00, A61P 17/04, A61P 17/06

(54) **TOPICAL CORTICOSTEROID COMPOSITIONS**
TOPISCHE CORTICOSTEROIDZUSAMMENSETZUNGEN
COMPOSITIONS DE CORTICOSTÉROÏDES TOPIQUES

(30) Priority: 11.03.2014 US 201461951165 P
(43) Date of publication of application: 18.01.2017
(73) Proprietor: Promius Pharma, LLC, Princeton, NJ 08540 (US)
(72) Inventor: KANDAVILLI, Sateesh, Plainsboro NJ 08536 (US); SAHUKAR, Priyadarshani, Hyderabad Andhra Pradesh 500090 (IN); OKUMU, Franklin, Oakland, CA 94611 (US)
(74) Representative: Henderson, Helen Lee
(86) International application number: PCT/US2015/020031
(87) International publication number: WO 2015/138650

(56) References cited:
- WO-A2-2006/003481
- WO-A2-2011/026076
- US-A1- 2011 305 643

## Description

### CROSS-REFERENCE TO RELATED APPLICATIONS

The present application claims the benefit of the filing date of U.S. Provisional Patent Application No. 61/951, 165 filed March 11, 2014.

### FIELD OF THE INVENTION

The present application relates to an aqueous based topical corticosteroid composition.

### BACKGROUND

Topical drug delivery systems are an ideal choice for treating various skin disorders locally. Topical dosage forms such as ointments, creams, gels, sprays, etc. are available to deliver the active agents to diseased area of the skin.

Inflammatory skin disorders are common in people of all age groups, races and genders, and these disorders are characterized by inflammation and irritation of the skin. Diagnosis and treatment of inflammatory skin disorders remains challenging in dermatological practice. Psoriasis is one of the inflammatory skin disorders. It is a chronic papulosquamous cutaneous disease which manifests through the appearance of red scaly patches on the skin. It generally affects the elbows, knees, and scalp. Although many therapeutic choices are available to treat psoriasis such as: topical therapy, phototherapy and systemic therapy, topical corticosteroids are the first choice for treating psoriasis. Phototherapy and systemic therapy are secondary and they are generally preferred when topical corticosteroids fail in treating psoriasis.

Corticosteroids are widely used in clinical practice. In particular, topical corticosteroids have been used to treat various skin conditions such as psoriasis, dermatitis, etc. Corticosteroids are chemically classified into hydrocortisone type, acetonide type, betamethasone type, etc. They are also classified based on their potency in the Vasoconstrictor assay (VCA), otherwise called the skin blanching assay.

The VCA is often used to access the potency of topically administered corticosteroids and to determine the bioequivalence of topically administered corticosteroids as U.S. Food and Drug Administration (FDA) guidance for industry. Accordingly, corticosteroids can be classified by VCA as super potent (Class 1), high potent (Class 2), upper mid strength (Class 3), mid strength (Class 4), lower mid strength (Class 5), low potent (Class 6), and least potent (Class 7).

The drug compound having the adopted name "betamethasone dipropionate" belongs to super potent (Class 1) and/or high potent (Class 2) and has a chemical name 9-fluoro-11(β),17,21-trihydroxy-16(β)-methylpregna-1,4-diene-3,20-dione 17,21-dipropionate and is represented by structural Formula I.

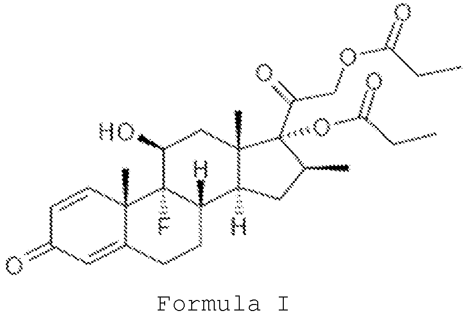

Betamethasone dipropionate is a white to cream white, powder. It is practically insoluble in water, sparingly soluble in ethanol and freely soluble in acetone and chloroform.

Topical betamethasone dosage forms, such as aerosol foam, cream, ointment, gel, and lotion formulations are commercially available. Combination formulations of betamethasone dipropionate with calcipotriene hydrate and also with clotrimazole exist. Betamethasone dipropionate is the active ingredient in commercially available products sold as DIPROLENE AF® and DIPROLENE® that comprise 0.05% betamethasone base, and are intended for application to affected skin areas once or twice daily.

Some topical corticosteroids are administered as occlusive dosage forms, which cause stratum corneum to hydrate thereby improving penetration of corticosteroidal drug into skin layers. The ointment dosage form has greater absorption because of the occlusive nature of the ointment base, however, which creates greasy sensation to subjects. Moreover, it is necessary to rub such formulations into the target site to improve the penetration of the active agent into the epidermis, an action which itself produces irritation. In addition, the presence of alcohol causes irritation/stinging to subject skin, and solution based topical compositions have tendency to evaporate before the active agent penetrates the epidermis. Propellant-containing topical aerosol compositions, in the market, are priced relatively higher than their counterparts.

The stratum corneum (SC) is the first layer of the skin comprising dead cells and provides the rate limiting step in percutaneous absorption of drugs through the skin layers. There are only a few drugs that possess the physiochemical properties to necessary to penetrate the SC. Percutaneous absorption involves the passage of the drug molecule from the skin surface into the stratum corneum under the influence of a concentration gradient and the drug molecule's subsequent diffusion through the stratum corneum and underlying epidermis, through the dermis.

The nature of the vehicle in a topical composition has a profound effect on the rate of release and delivery of the agent in the skin layers passage through the stratum corneum. The vehicle used to deliver the drug can aid in the efficacy and stability of the product. Generally aqueous vehicles are preferred in topical dosage form due to their non-irritant, superior tolerability and non-toxic nature. An important aspect here is that most of corticosteroid drugs are exceptionally poorly soluble in aqueous vehicles and cause instability.

Another important aspect in a topical composition is the inclusion of a substance which assists the active agent to penetrate or diffuse through the skin layers, i.e., "skin penetration enhancers." Skin penetration enhancers are necessary for the active to penetrate in the skin layers. Various classes of skin penetration enhancers are available, such as, fatty acids and their esters, pyrrolidones, sulfoxides, glycols, glycerides, etc. However, skin penetration enhancers are known to act differently with different active agent.

Conventionally, topical coricosteroid products are available as creams, lotions and ointments. U.S. Patent No. 3,892,856 describes the use of corticosteroids dissolved in polyethylene glycol and emulsified into an oleaginous base.

U.S. Patent 3,934,013 describes topical pharmaceutical compositions containing at least two corticosteroids, propylene glycol, a fatty alcohol and water. The patentee describes the "fatty alcohol ingredient" as any fatty alcohol having from 16-24 carbon atoms and, preferably, as a saturated, monohydric primary alcohol such as cetyl alcohol, stearyl alcohol or behenyl alcohol.

U.S. Patent 4,343,798 discloses topical antimicrobial/anti-inflammatory compositions containing C₅-C₁₂ fatty acids in combination with corticosteroids.

PCT application WO 2011/026076 discloses pharmaceutically topical sprayable compositions comprising steroid as active agent.

U.S. Patent Nos. 6,126,920 and 7,078,058 disclose betamethasone valerate aerosols with a quick-break foaming agent, a propellant, and a buffering agent, wherein ethanol is present.

U.S. Patent No. 5,369,131 discloses a liquid mechanically foamable pharmaceutical composition, which is propellant free, for local application.

U.S. Patent Application Publication No. 2008/0102039 discloses spray foaming dosage compositions comprising propylene glycol.

U.S. Patent No. 5,958,379 discloses a pharmaceutical composition that is sprayable as liquid droplets, forming a preparation within times less than 4 seconds.

U.S. Patent Application Publication No. 2006/0239929 discloses a sprayable composition for the treatment of psoriasis, comprising clobetasol as the active agent together with ethyl alcohol.

US 2011/305643 and WO 2006/003481, both, disclose sprayable, topical bethamethasone dipropionate compositions which comprise oleyl alcohol, water and other excipients.

There is an unmet need for subject compliant topical formulations that are effective in the treatment of skin disorders such as psoriasis, and which provide improved delivery of the active agent at the desired site of action, with decreased inconvenience and irritation, increased ease of use for the subject, and longer duration of action. Topical spray compositions are always preferred over any other topical dosage forms due to subject acceptance and convenience of application in skin area.

Topical corticosteroids are widely approved for use in various skin disorders and topical corticosteroids are known to have solubility issues such that corticosteroids are insoluble in water or aqueous solvents. The propylene glycol is an essential solvent and/or cosolvent in the topical compositions containing corticosteroids. It is widely known for solubilizing corticosteroids and acts as cosolvent to facilitate solubility of corticosteroids in the topical compositions. Furthermore propylene glycol is known as better skin penetration enhancer for corticosteroids. Propylene glycol is used in more than 100 approved topical compositions comprising corticosteroids. On the other hand propylene glycol causes significant allergy and skin irritation to the subject's skin.

Aqueous based topical spray composition of the present application is formulated to achieve equal or superior efficacy to marketed products and to overcome the shortcomings of subject compliance in the use of topical dosage forms.

### SUMMARY OF THE INVENTION

The present application relates to an aqueous based topical spray composition comprising: a) a corticosteroid which is a betamethasone compound; b) a fatty alcohol acting as a skin penetration enhancer, wherein the fatty alcohol comprises oleyl alcohol and is present in an amount of about 0.001% to about 15% by weight based on the total weight of the composition; c) at least one pharmaceutically and/or dermatologically acceptable excipient; and d) water ; and e) wherein the composition is free of propylene glycol, free of propellant and substantially non-foaming, such that the topical spray composition forms mist or droplet in more than 90% of quantity when sprayed onto the affected skin area.

Another aspect of the present application relates to said aqueous based topical spray compositions for use in prophylaxis, amelioration, or treatment of psoriasis, corticosteroid responsive dermatoses, erythema, contact sensitivity reactions, and other associated diseases or disorders.

Another aspect of the present application which is not claimed, relates to a process for preparing an aqueous based topical spray composition, comprising:
**a)** heating a mixture comprising an emulsifying agent and a water-immiscible substance to obtain an oily phase;
**b)** optionally, mixing an antioxidant, preservative, or both with the oily phase of a);
**c)** mixing an active agent with a penetration enhancer;
**d)** mixing the material of c) with the mixture of a) or b);
**e)** dissolving a polymer in water to form an aqueous phase; and
**f)** mixing the oily phase of d) with an aqueous phase of e), to form an emulsion.

### BRIEF DESCRIPTION OF THE DRAWINGS

Figure 1 shows the structures of certain betamethasone propionate impurities.

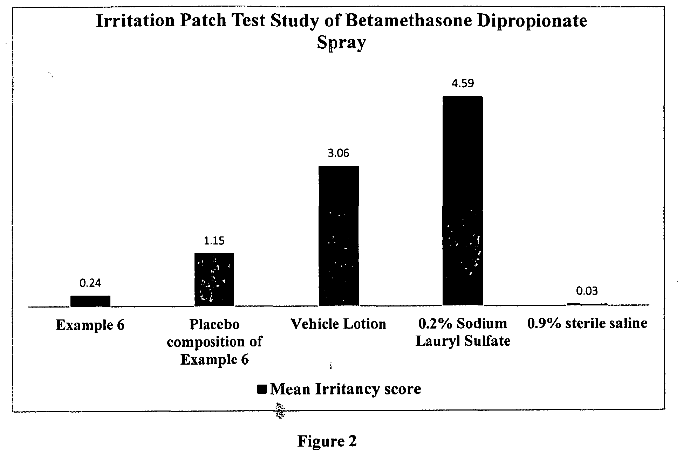

Figure 2 shows mean irritation score of Example 6 in comparison with other vehicles in Irritation Patch Test Study of betamethasone dipropionate spray.

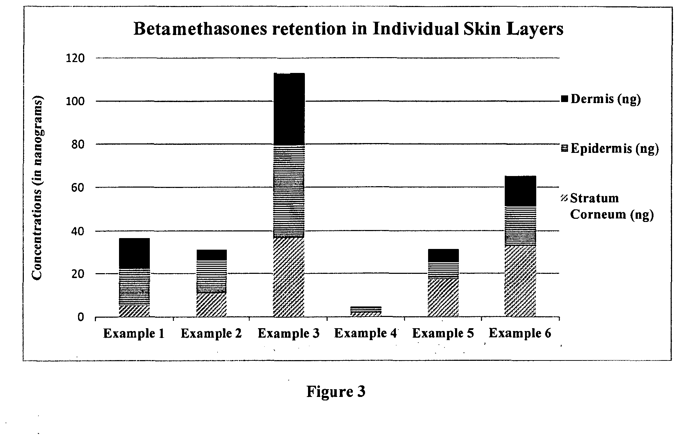

Figure 3 shows amounts of betamethasones retention in individual skin layers by Examples 1-6.

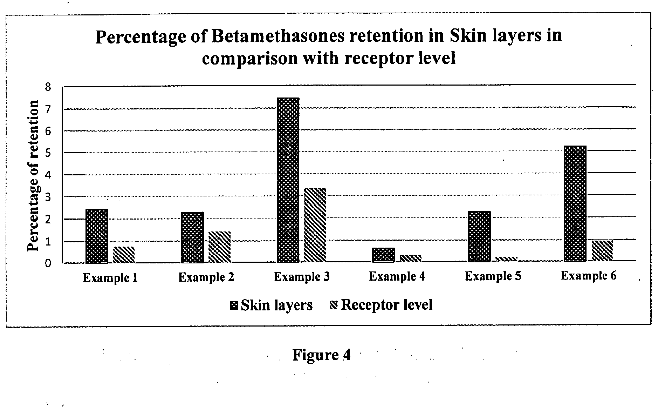

Figure 4 shows percentage of betamethasones retention in skin layers in comparison with receptor level by Examples 1-6.

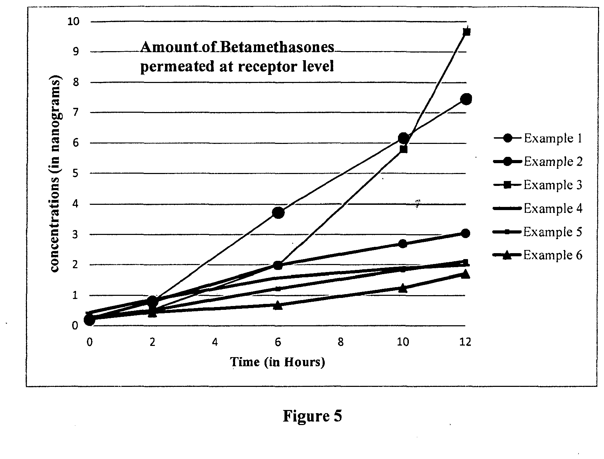

Figure 5 shows amounts of betamethasones permeated at receptor level by Examples 1-6.

### DETAILED DESCRIPTION OF THE INVENTION

The term "stable" as used herein refers to physical stability and/or chemical stability of the active agent in a topical composition, wherein changes in the drug assay values and/or impurities content are less than about 10%, during stability study storage of the composition at 25°C and 60% relative humidity (RH), or 30°C and 65% RH, or 40°C and 75% RH, for durations such as 3, 6, 12, 18 or 24 months.

The term "propellant free" or "free of propellant(s)" as used herein indicates that the compositions are not delivered using any of the commonly used aerosol propellants, such as fluorochloro hydrocarbons, hydrocarbons, compressed gases, and the like.

The term "substantially free" as used herein indicates that the specified substance referred to is present in amounts not more than 10% by weight of the total composition.

The term "substantially non-foaming" as used herein indicates that the topical spray composition forms mist or droplet in more than 90% of quantity, when sprayed on to the affected skin area. Preferably, the topical spray composition forms mist or droplet in more than 95% of quantity, when sprayed on to the affected skin area.

The term "substantially non-irritating" as used herein indicates that an aqueous based topical spray compositions of the present application does not cause erythema, papules, definite edema, vesicular eruption at test site, and any noticeable strong reaction which is spreading beyond test site even in semi occlusive conditions.

A "skin permeation enhancer" or "skin penetration enhancer" or "penetration enhancer" is a component used to enhance the penetration rate of drugs through the skin or mucous membrane, such as by temporarily diminishing the impermeability of the skin or membrane. Permeation enhancers have also been called "accelerants" and "absorption promoters." There are numerous penetration enhancers that can be used. It has been found that when fatty alcohols reduce permeation lag time thereby enhancing the delivery into epidermis and dermis. In an aspect of the present application, the skin penetration enhancer, without any limitation, is selected from C₅-C44 fatty alcohols, preferably C₅-C20 fatty alcohols. These fatty alcohols belong to the group of long chain saturated fatty alcohols, unsaturated chain fatty alcohol, branched chain alcohol or combinations thereof.

"Emollients" are substances that soften and soothe the skin. They are used to correct dryness and scaling of the skin. Various emollients include, but are not limited to, oils of natural origin such as almond oil, coconut oil, olive oil, palm oil, peanut oil and the like, fatty acids such as lauric acid, myristic acid, palmitic acid, and stearic acid, monohydric alcohol esters of the fatty acids such as ethyl laurate, isopropyl laurate, ethyl myristate, n-propyl myristate, isopropyl myristate, ethyl palmitate, isopropyl palmitate, methyl palmitate, methyl stearate, ethyl stearate, isopropyl stearate, butyl stearate, isobutyl stearate, amyl stearate, and isoamyl stearate, glycols such as ethylene glycol, diethylene glycol, and polyethylene glycol, mineral oil and any combinations thereof.

The term "aqueous based" as used herein indicates that the carrier of the topical spray composition comprises a majority of water in the composition. In an aspect, the term "aqueous based" as used herein denotes that the said topical spray composition comprising at least about 60%w/w or at least about 70% w/w of water based on total weight of the composition. The term "carrier" denotes organic or inorganic ingredients, natural or synthetic, with which an active ingredient is combined to facilitate application of a composition. In the present context, the terms "carrier" and "vehicle" are interchangeably used. The term "carrier" includes, but is not limited to, water, acetone, alone or in combination with materials such as silicone fluids. The amounts of carrier may be about 5% to about 99% of the total weight of the composition. In embodiments, a carrier according to the present application comprises water. In embodiments, the carrier can comprise, in addition to water, water-immiscible substances such as any pharmaceutically and/or dermatologically acceptable fatty esters of natural fatty acids, triglycerides of animal or vegetable, medium chain triglycerides, mixtures of mono-, di- and/or triglycerides, waxes, hydrogenated vegetable oils, and mixtures thereof.

The term "preservative" refers to a natural or synthetic chemical that is added to products to prevent decomposition by microbial growth or by undesirable chemical changes. Preservatives can desirably be incorporated into a composition for protecting against the growth of potentially harmful microorganisms. While microorganisms tend to grow in an aqueous phase, microorganisms can also reside in a hydrophobic or oil phase. Suitable preservatives for compositions of the present application include, but are not limited to, methylparaben, propylparaben, benzyl alcohol, chlorocresol, benzalkonium chloride, cetrimonium chloride, sodium edetate, boric acid, and any mixtures thereof. The amount of preservative may be from about 0.25% to about 25% of the total weight of the composition.

The term "betamethasone compound" represents, but is not limited to, betamethasone base, betamethasone dipropionate, betamethasone valerate, betamethasone acetate, betamethasone benzoate, betamethasone dipropionate, betamethasone sodium phosphate, betamethasone valerate, betamethasone sodium phosphate, betamethasone 17-propionate, betamethasone 21-propionate, and betamethasone 17-propionate 21-acetate and/or mixtures thereof. Unless otherwise specified, a betamethasone compound is intended to include its isomers, its salts or its esters.

The term "betamethasones" represents betamethasone dipropionate, betamethasone 17-propionate, betamethasone 21-propionate, betamethasone base and/or mixtures thereof.

The term "corticosteroid" represents a compound selected from the group comprising of: alclometasone dipropionate, amcinonide, beclomethasone dipropionate, betamethasone base, betamethasone benzoate, betamethasone dipropionate, betamethasone sodium phosphate, betamethasone valerate, betamethasone 17-monopropionatye, betamethasone 21-monopropionate, budesonide, clobetasol propionate, clobetasone butyrate, clocortolone pivalate, desonide, desoximetasone, dexamethasone, dexamethasone acetate, dexamethasone nicotinate, dexamethasone propionate, dexamethasone sodium phosphate, dexamethasone valerate, diflorasone diacetate, diflucortolone valerate, fluandrenolide, flumethasone pivalate, fluocinolone acetonide, fluocinonide, fluocortin butyl ester, fluticasone propionate, halcinonide, halobetasol propionate, halometasone monohydrate, hydrocortisone, hydrocortisone sodium phosphate, hydrocortisone sodium succinate, hydrocortisone-17-butyrate-21-propionate, hydrocortisone aceponate, hydrocortisone acetate, hydrcortisone valerate, hydrocortisone butyrate, hydrocortisone probutate, methylprednisolone, methylprednisolone acetate, methylprednisolone aceponate, mometasone furoate, prednisolone, prednisolone sodium phosphate, prednisolone acetate, prednisolone-17-valerate-21-acetate, triamcinolone acetonide, triamcinolone acetate, triamcinolone diacetate, and prednicarbate. Unless otherwise specified, recitation of corticosteroid or specific compound is intended to include the specific compound or any salts, esters, isomersmetabolites, conjugates, derivatives or prodrugs thereof.

"Solvent" refers to components that aid in the dissolution of the drug in the composition. Solvents serve to maintain a solution of the drug in the composition. Some solvents can also enhance percutaneous penetration of drug and/or act as humectants. For corticosteroid drugs, solvents can include water-immiscible substances such as fatty esters of natural fatty acids, triglycerides of animal or vegetable, medium chain triglycerides, mixtures of mono-, di- and/or triglycerides, waxes, hydrogenated vegetable oils, and mixtures thereof. Some specific examples include castor oil, lanolin oil, citrate triisocetyl triglycerides having 10-18 carbon atoms, caprylic/capric triglycerides, coconut oil, corn oil, cottonseed oil, linseed oil, oil of mink, olive oil, palm oil, sunflower oil, nut oil, diethylene glycol monoethyl ether, diethylene glycol monomethyl ether, saturated paraffin oils, light or heavy mineral oils, vegetable oils, glycerides, and the like, and/or their mixtures thereof. The term "applied dose" as used herein means the amount of topical spray composition dispensed to the subject skin in one actuation of topical spray device. For example, if an applied dose is about 170 mg which contains about 0.05%w/w of betamethasone compound (about 0.085 mg), the percentage retention of betamethasone compound in skin layer is about 0.1% to about 10% of 0.085 mg of betamethasone(s).

The term "skin depot" as used herein refers to a topical composition which provide higher skin retention of the applied drug compared to systemic exposure of the same drug. The term "skin layers" as used herein includes stratum corneum, epidermis and dermis of mammalian skin. The term "systemic exposure" as used herein includes tendency of any topically applied drugs entering into systemic circulation of mammals, thereby causing systemic side effects, such as corticosteroids causes systemic side effects of hypothalamic-pituitary-adrenal axis suppression.

Various aspects of the present application relate to an aqueous based topical spray composition comprising a corticosteroid as defined by the claims.

An aspect of the present invention relates to an aqueous based topical spray composition, according to claim 1, which *inter alia* comprises: a) a corticosteroid; b) at least one fatty alcohol; c) at least one pharmaceutically and/or dermatologically acceptable excipient(s); and d) water.

The fatty alcohol in the above composition is acting as a skin penetration enhancer or a skin permeation enhancer.

In another aspect, the fatty alcohol is C₅-C20 fatty alcohols.

In another aspect, these fatty alcohols selected from saturated fatty alcohols, unsaturated fatty alcohols, branched chain fatty alcohols and mixutures thereof.

In another aspect, the fatty alcohol is selected from the group comprising of, but not limited to, elaidyl alcohol, linoleyl alcohol, linolenyl alcohol, caproic alcohol, lauryl alcohol, stearyl alcohol, cetostearyl alcohol, behenyl alcohol, oleyl alcohol, 2-heptyl-1-undecanol, 1, 17-heptadecanediol, and combinations thereof.

In another aspect, the composition is oil-in-water emulsion.

In another aspect, the composition is substantially free of (C₁-C₄) alcohol.

In another aspect, the composition is free of propellants.

An aspect of the present application relates to the composition, which delivers same or more amount of the corticosteroid in the skin layers compared to DIPROLENE Lotion Augmented, 0.05%.

In another aspect, the composition of the present application is non-irritating to the skin, non-toxic and well-tolerated.

According to the invention, the corticosteroid present in the composition of the present application is a betamethasone compound.

In another aspect, the betamethasone compound is selected from the group comprising of betamethasone benzoate, betamethasone dipropionate, betamethasone sodium phosphate, betamethasone valerate, and combinations thereof.

In another aspect, the betamethasone compound is in the form of betamethasone dipropionate.

In another aspect, which is not claimed, the corticosteroid present in the composition of the present application is mometasone furoate.

In another aspect, the corticosteroid present in the composition of the present application is betamethasone valerate.

In another aspect, which is not claimed, the corticosteroid present in the composition of the present application is triamcinolone acetonide.

In another aspect, which is not claimed, the corticosteroid present in the composition of the present application is alclometasone dipropionate.

An aspect of the present application relates to the aqueous based topical spray composition claimed for use in prophylaxis, amelioration, or treatment of psoriasis, corticosteroid responsive dermatoses, erythema, contact sensitivity reactions, and other associated diseases or disorders.

An aspect of the present invention relates to the aqueous based topical spray composition claimed for use in prophylaxis, amelioration, or treatment of moderate to severe plaque psoriasis.

An aspect of the present invention relates to the aqueous based topical spray composition claimed for use in prophylaxis, amelioration or treatment of moderate plaque psoriasis.

In another aspect, the concentration of the corticosteroid in the composition of the present application is from about 0.01% to about 10%, or from about 0.025% to about 5%, or from about 0.025% to about 0.5%, by weight based on the total weight of the composition.

A specific aspect of the application relates to an aqueous based topical spray composition comprising a betamethasone compound, in amounts equivalent to about 0.025 to about 0.1 percent by weight of betamethasone base.

According to the invention, the fatty alcohol is present in an amount of about 0.001% to about 15% percent by weight based on the total weight of the composition.

An aspect of the present invention relates to an aqueous based topical spray composition as defined in claim 1 comprising: a corticosteroid, a skin penetration enhancer, an emulsifying agent, a polymer, water, and a water-immiscible substance, wherein the skin penetration enhancer is present in the amount of about 0.001% to about 15% percent by weight based on the total weight of the composition.

In another aspect, the skin penetration enhancer is present in an amount of about 0.05% to about 12%, specifically about 3% to about10% by weight based on the total weight of the composition.

In a further aspect of the present application, which is not claimed, an aqueous based topical spray composition comprises: a) betamethasone dipropionate; b) at least one fatty alcohol; c) at least one pharmaceutically and/or dermatologically acceptable excipient; and d) water, wherein said the fatty alcohol is selected from elaidyl alcohol, linoleyl alcohol, linolenyl alcohol, caproic alcohol, lauryl alcohol, stearyl alcohol, cetostearyl alcohol, behenyl alcohol, oleyl alcohol, 2-heptyl-1-undecanol, 1,17-hepatadecanediol and mixtures thereof, and wherein said topical spray composition is substantially free of (C₁-C₄)alcohol and free of propellants.

In another aspect of the present application, an aqueous based topical spray composition as defined in claim 1 comprises: a) betamethasone dipropionate; b) an oleyl alchol; c) at least one pharmaceutically and/or dermatologically acceptable excipient; and d) water.

In another aspect, the composition further comprises emulsifying agent.

In another aspect, the composition does not form any film layer.

In another aspect, the composition is oil-in-water emulsion,

In another aspect, the above composition is substantially free of (C₁-C₄)alcohol.

The composition is free of propellants.

An aspect of the present application relates to aqueous based topical spray composition as claimed for use in prophylaxis, amelioration, or treatment of psoriasis, corticosteroid responsive dermatoses, erythema, contact sensitivity reactions, and other associated diseases or disorders.

Another aspect of the present application relates to the above composition for use in prophylaxis, amelioration, or treatment of moderate plaque psoriasis.

Another aspect of the present application, which is not claimed, relates to a process for preparing a topical spray composition, comprising:
**a)** heating a mixture comprising an emulsifying agent and a water-immiscible substance to obtain an oily phase;
**b)** optionally, mixing an antioxidant, preservative, or both with the oily phase of a);
**c)** mixing an active agent with a penetration enhancer;
**d)** mixing the material of c) with the mixture of a) or b);
**e)** dissolving a polymer in water to form an aqueous phase; and
**f)** mixing the oily phase of d) with an aqueous phase of e), to form an emulsion.

In further aspect, an aqueous based topical spray composition of the present application is substantially non-irritating to the skin, non-toxic and well-tolerated, thereby providing a high degree of subject compliance, and is useful in the prophylaxis, amelioration or treatment of skin diseases or disorders such as psoriasis, steroid responsive dermatoses, erythema, contact sensitivity reactions, and other associated diseases or disorders.

In another aspect, composition of the present application relates to sustained release of the corticosteroid, for better skin permeation and subject comfort.

In an aspect, the present application provides a method of using the claimed propellant-free topical spray composition comprising at least one corticosteroid as an active agent, wherein the method comprising administering a pharmaceutically and/or dermatologically effective amount of a spray composition directly onto an affected part of the skin of a subject in need thereof.

In another aspect, topical spray composition of the present application is useful in the management of psoriasis, and further can provide a moisturizing and/or soothing effect at the site of application to the skin.

In another aspect, the composition reduces the dryness that accompanies the build-up of skin in psoriatic plaques.

In another aspect, the composition of the present application can be applied directly to the psoriatic lesions or dermatoses and can help reduce inflammation, remove built-up scale, reduce skin turnover, and/or clear affected skin of plaques.

Vasoconstriction assay (VCA) is used to measure dermatological potency of the topical corticosteroids and it recommended method to access *in vivo* bioequivalence of topical corticosteroid by US FDA *(ref: Guidance for industry; Topical dermatological corticosteroids: in vivo Bioequivalence; Dated June 02, 1995)* .

VCA study is performed *in vivo* and results are obtained based on blanching effect of the skin by two methods, one is chromameter method and the other one is visual scoring method. VCA is often considered for accessing potency, however, the result of the VCA study depends on the concentration of drug in stratum corneum and epidermis.

Topical spray composition of the present application makes drug available in the dermis layer of the skin thereby exhibiting equal or more potent than the marketed dosage form (DIPROLINE Lotion Augmented, 0.05%).

The fatty alcohols contain at least one primary alcohol group in long chain hydrocarbons (C₅-C₄₄) and are derived from natural sources as well as synthetically made from fatty acids. Fatty alcohols are widely used in cosmetic and pharmaceutical industry in the preparation of topical drug compositions and cosmetic preparations such as hair care products, conditioners etc. Fatty alcohols are used as emollients, skin penetration enhancers, emulsifiers and thickeners. Unsaturated fatty alcohols contain, in addition to the primary alcohol group, at least one olefinic group in the chain and additionally they have "Z" (cis) and "E" (trans) configuration at the olefinic group in the chain. The physical and chemical properties of the fatty alcohols greatly vary depending on length of the chain and/or the presence or absence of the olefinic group in the chain. The selection and usability of the fatty alcohols depend mainly on the choice of active agent since fatty alcohols are known to act differently with different active agents due the chemical nature of the active agent. In another aspect, the fatty alcohols contain at least one primary alcohol group in long chain hydrocarbons (C₅-C₂₀). According to the invention, the fatty alcohol comprises oleyl alcohol.

In an aspect of the invention, the skin penetration enhancer is selected from the group comprising of elaidyl alcohol, linoleyl alcohol, linolenyl alcohol, caproic alcohol, lauryl alcohol, stearyl alcohol, cetostearyl alcohol, behenyl alcohol, oleyl alcohol, 2-heptyl-1-undecanol, 1,17-heptadecanediol and mixtures thereof.

In another aspect of the present application, the skin penetration enhancer is branched chain fatty alcohol which is selected from 2-methyl-1-pentanol, 2-ethyl-hexanol, 2-propyl-heptanol, 2-butyl-octanol, 2-pentyl-1-nonanol, 2-hexyl-1-decanol, 1,6-hexanediol, 1,7-heptanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol and mixtures thereof.

In another aspect, the skin penetration enhancer is selected from unsaturated fatty alcohols.

In another aspect, the skin penetration enhancer is selected from unsaturated fatty alcohol having at least one unsaturation bond in the fatty alcohol chain and having "Z" configuration. In another aspect, oleyl alcohol is a skin penetration enhancer in the context of present application.

In another aspect, the composition of the present application comprises one or more additional active agents useful in the management of psoriasis and associated pathological conditions including synthetic, semi-synthetic, or naturally obtained active agents. The compositions of the present application can be used for prophylaxis, amelioration, or treatment of skin diseases and disorders, by administration of a pharmaceutically and/or dermatologically effective amount of the spray composition to a subject in need thereof. The compositions of the present application are also useful in conjunction with other therapies, such as phototherapy.

In another aspect, the composition of the present application is easily water-washable and removable from the site of application.

In another aspect, the composition of the present application, when applied by spraying onto the skin, are substantially non-occlusive to the skin and does not form any film layer/residues at the site of application.

The compositions of the present application are free of propylene glycol.

In another aspect, the composition of the present application is substantially free of (C₁-C₄) alcohols and propylene glycol, such that any amounts present do not cause significant skin irritation or impart any undesired attributes to the composition.

The composition of the present application is substantially non-foaming, free of propylene glycol and free of propellant.

In another aspect, the composition of the present application does not cause significant skin irritation even in occlusive condition.An aqueous based topical spray composition of the present application is free of propylene glycol and stable for at least for the period of about 6 months at 40°C or at least for the period of 24 months at 25°C.

Another aspect of the present application, which is not claimed, provides dispensing devices for the topical delivery of the compositions onto the skin in the form of sprays. In another aspect, which is not claimed, the present application provides devices, into which the composition is filled, comprising a container, a dispenser, and a closure.

Another aspect of the present application, which is not claimed, relates to a dispensing device containing propellant-free topical composition, wherein a device comprises a container, a pump dispenser, a dip tube, a metering valve, and an actuator, and wherein the pump dispenser is capable of dispensing the composition through a dip tube into a metering valve, and through the actuator fitted with an orifice, such that the composition is consistently released in the form of a substantially uniform spray.

An aspect of the present application, which is not claimed, relates to dispensing device containing a propellant-free topical composition, wherein the device comprises a container having therein a pouch system or bag filled with the composition, optionally fitted with a dip tube and an actuator fitted with a valve, the container being filled with a gas such as nitrogen gas or compressed air, surrounding the pouch or bag. Introduction of the composition into the system can further increase the pressure of the system, which is capable of dispensing the composition from the pouch or bag into the actuator fitted with a valve, such that the composition is released upon actuation in the form of a spray.

In another aspect, advantages of topical sprayable composition of the present application include non-irritancy to the site of application, ease of application, usefulness for long periods, non-staining of fabrics, and not possessing a strong or objectionable odor. This facilitates a subject in need thereof to maintain regular applications of the medications, and thus avoids abrupt withdrawal of the corticosteroid composition application, which in turn prevents an aggressive recurrence of the disease condition.

In another aspect, a corticosteroid present in the topical compositions is betamethasone dipropionate, which typically is administered in doses of about 0.001 mg/kg body weight to about 0.5 mg/kg body weight, to a subject in need thereof.

In another aspect, the compositions of the present application may be in the form of solutions, suspensions, emulsions, lotions, microemulsions, nanoemulsions, emulgels, gels, and the like. In embodiments, compositions may be in the form of an emulsion. The emulsion can be in the form of an oil-in-water type of emulsion or a water-in-oil type of emulsion. An aqueous-based emulsion, such as an oil-in-water emulsion, frequently has lower viscosity than other emulsion types and exhibits appreciable storage stability. Generally, oil-in-water emulsions have better skin feel properties, when applied to the skin, as these give sensations similar to an aqueous material. When the oily phase is dispersed as droplets within an aqueous continuous phase, this is called an "oil-in-water" type of emulsion. When an aqueous phase is dispersed as droplets within an oily continuous phase, this is called a "water-in-oil" type of emulsion. In embodiments, the hydrophobic phase comprises about 0.5% to about 90% by weight of the composition. Compositions in the form of emulsions may be micro- or nanoemulsions. In embodiments, average particle sizes of the dispersed phase droplets are less than about 500 pm. In embodiments, average particle sizes of the dispersed phase droplets are less than about 2000 nm.

In another aspect, the compositions of the present application are formulated as emulsions, comprising an oily or hydrophobic phase, an aqueous or hydrophilic phase, and an emulsifier.

The compositions of the present application include pharmaceutically and/or dermatologically acceptable excipients including, but not limited to, one or more of carriers, emulsifiers, coemulsifiers, permeation or penetration enhancers, solvents, co-solvents, emollients, antioxidants, preservatives, buffering agents, gelling or thickening agents, polymers, surfactants, soothing agents, pH modifiers, solubilizers, humectants, emollients, moisturizers, oily bases, and the like.

Examples of suitable polymers for use in the present application include, but are not limited to carbomers, polyethylene glycols, acrylate polymers, methacrylate polymers, polyvinylpyrrolidones, copolymers based on butyl methacrylate and methyl methacrylate povidone, vinyl acetates, polyvinyl acetates, celluloses, gums, alginates, cellulose acetate phthalates, cellulose acetate butyrates, hydroxypropyl methyl cellulose phthalates, and the like. Examples include Carbopol® products, PEG 400, Eudragit® 100, Eudragit® RSPO, Eudragit® RLPO, Eudragit® ND40, Plasdone®, copolymers based on butyl methacrylate and methyl methacrylate (Plastoid® B), alkyl celluloses such as ethyl celluloses and methyl celluloses, hydroxyalkyl celluloses such as hydroxyethyl cellulose and hydroxypropyl cellulose, hydroxyalkyl alkyl celluloses such as hydroxypropyl methylcelluloses and hydroxybutyl methylcelluloses, gums such as xanthan gum, tragacanth, guar gum, locust bean gum, acacia, and the like.

Other polymers that are useful include polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyglycolides, polysiloxanes, polyurethanes and copolymers thereof, cellulose ethers, cellulose esters, nitrocelluloses, polymers of acrylic and methacrylic esters, cellulose acetates, cellulose propionates, cellulose acetate butyrates, cellulose acetate phthalates, carboxylethyl celluloses, cellulose triacetates, cellulose sulphate sodium salts, poly(methyl ethacrylate), poly(ethylmethacrylate), poly(butylmethacrylate), poly(isobutylmethacrylate), poly(hexylmethacrylate), poly(isodecylmethacrylate), poly(lauryl methacrylate), poly(phenyl methacrylate), poly(methyl acrylate), poly(isopropyl acrylate), poly(isobutyl acrylate), poly(octadecyl acrylate), polyethylenes, polypropylenes, poly(ethylene glycol), poly(ethylene oxide), poly(ethylene terephthalate), poly(vinyl alcohol), poly(vinyl acetate), poly(vinyl chloride), polystyrenes, and the like, including any mixtures thereof.

Further useful polymers include synthetic polymers, such as polymers of lactic acid and glycolic acid, polyanhydrides, poly(ortho ester), polyurethanes, poly(butyric acid), poly(valeric acid), poly(caprolactone), poly(hydroxybutyrate), poly(lactide-co-glycolide), poly(lactide-co-caprolactone), and natural polymers such as alginate and other polysaccharides that include but are not limited to arabinans, fructans, fucans, galactans, galacturonans, glucans, mannans, xylans (such as, for example, inulin), levan, fucoidan, carrageenan, galatocarolose, pectic acid, pectin, amylose, pullulan, glycogen, amylopectin, cellulose, dextran, pustulan, chitin, agarose, keratan, chondroitan, dermatan, hyaluronic acid, alginic acid, xanthan gum, starches, and various other natural homopolymers and heteropolymers, such as those containing one or more of aldoses, ketoses, acids or amines, erythrose, threose, ribose, arabinose, xylose, lyxose, allose, altrose, glucose, mannose, gulose, idose, galactose, talose, erythrulose, ribulose, xylulose, psicose, fructose, sorbose, tagatose, mannitol, sorbitol, lactose, sucrose, trehalose, maltose, cellobiose, glycine, serine, threonine, cysteine, tyrosine, asparagine, glutamine, aspartic acid, glutamic acid, lysine, arginine, histidine, glucuronic acid, gluconic acid, glucaric acid, galacturonic acid, mannuronic acid, glucosamine, galactosamine, and neuraminic acid, and naturally occurring derivatives thereof, and including dextran and cellulose, collagen, albumin and other hydrophilic proteins, zein and other prolamines and hydrophobic proteins, copolymers or mixtures thereof.

In further aspects, the amount of polymer may be about 0.001% w/w to about 45% w/w of the total weight of the composition. In an embodiment, the amount of polymer may be about 0.01% w/w to about 5% w/w of the total weight of the composition. In an embodiment, the amount of polymer may be about 0.05% w/w based on total weight of the composition.

Examples of suitable emulsifying agents include, but are not limited to, disodium cocoampho diacetate, oxyethylenated glyceryl cocoate (7 EO), PEG 30 Dipolyhydroxy stearate (Cithrol DPHS), Polyglyceryl-3 Diisostearate, PEG-20 hexadecenyl succinate, PEG-15 stearyl ether, Polyoxyl 20 Cetostearyl Ether, Polypropylene Glycol (PPG)-Stearyl Ether such as PPG-11 Stearyl Ether and PPG-15 Stearyl Ether, Polyoxypropylene Stearyl Ether (Arlamol E), ricinoleic monoethanolamide monosulfosuccinate salts, oxyethylenated hydrogenated ricinoleic triglyceride containing 60 ethylene oxide units such as the products sold by BASF under the trademarks Cremophor® RH 60 or Cremophor® RH 40 (polyoxyl 40 hydrogenated castor oil), polymers such as poloxamers, which are block copolymers of ethylene oxide and propylene oxide, and the nonsolid fatty substances at room temperature (that is to say, at temperatures ranging from about 20 to 35°C) such as sesame oil, sweet almond oil, apricot stone oil, sunflower oil, octoxyglyceryl palmitate (or 2-ethylhexyl glyceryl ether palmitate), octoxyglyceryl behenate (or 2-ethylhexyl glyceryl ether behenate), dioctyl adipate, and tartrates of branched dialcohols. Sorbitan fatty acid esters are a series of mixtures of partial esters of sorbitol and its mono- and dianhydrides with fatty acids. Sorbitan esters include products sold as Arlacel® 20, Arlacel 40, Arlacel 60, Arlacel 80, Arlacel 83, Arlacel 85, Arlacel 987, Arlacel C, PEG-6 stearate and glycol stearate and PEG-32 stearate (Tefose® 63), and PEG-6 stearate and PEG-32 stearate (Tefose® 1500), and any mixtures thereof. Polyethylene glycol ethers of stearic acid are in another group of emulsifiers that can be used in the emulsions. Examples of polyethylene glycol ethers of stearic acid are steareth-2, steareth-4, steareth-6, steareth-7, steareth-10, steareth-11, steareth-13, steareth-15, steareth-20, polyethylene glycol ethers of stearyl alcohol (steareth 21), and any mixtures thereof. Other emulsifying agents include sodium lauryl sulphate, cetyl trialkyl ammonium bromide, polyoxyethylene sorbitan fatty acid esters or any mixtures thereof.

Nonionic emulsifying agents include those that can be broadly defined as condensation products of long chain alcohols, e.g., C8-30 alcohols, with sugar or starch polymers, *i.e.,* glycosides. Various sugars include, but are not limited to, glucose, fructose, mannose, and galactose, and various long chain alcohols include, but are not limited to, decyl alcohol, cetyl alcohol, stearyl alcohol, lauryl alcohol, myristyl alcohol, oleyl alcohol, and the like.

Other useful nonionic emulsifying agents include condensation products of alkylene oxides with fatty acids such as alkylene oxide esters of fatty acids. Other nonionic surfactants are the condensation products of alkylene oxides with two moles of fatty acids such as alkylene oxide diesters of fatty acids.

Emulsifying agents can also include any of a wide variety of cationic, anionic, zwitterionic, and amphoteric surfactants that are known in the art. Non-limiting examples of anionic emulsifying agents include alkyl is ethionates, alkyl and alkyl ether sulfates and salts thereof, alkyl and alkyl ether phosphates and salts thereof, alkyl methyl taurates, and soaps (*e.g.,* alkali metal salts and sodium or potassium salts) of fatty acids.

Examples of amphoteric and zwitterionic emulsifying agents include those which are broadly described as derivatives of aliphatic secondary and tertiary amines in which the aliphatic radical can be straight or branched chain, wherein one of the aliphatic substituents contains from about 8 to about 22 carbon atoms and one contains an anionic water solubilizing group, e.g., carboxy, sulfonate, sulfate, phosphate, or phosphonate. Specific examples include alkylimino acetates, iminodialkanoates and aminoalkanoates, imidazolinium and ammonium derivatives. Other suitable amphoteric and zwitterionic emulsifying agents include betaines, sultaines, hydroxysultaines, alkyl sarcosinates, and alkanoyl sarcosinates.

Silicone emulsifying agents are typically organically modified organopoly siloxanes, sometimes called silicone surfactants. Useful silicone emulsifying agents include dimethicone copolyols. These materials are polydimethyl siloxanes, which have been modified to include polyether side chains such as polyethylene oxide chains, polypropylene oxide chains, mixtures of these chains, and polyether chains containing moieties derived from both ethylene oxide and propylene oxide.

The amounts of emulsifier may be from about 0.25% to about 45% of the total weight of the composition.

Co-emulsifiers or secondary emulsifying agents include polyoxylglycerides such as oleoyl macrogolglycerides (Labrafil® M 1944CS), linoleoyl macrogolglycerides (Labrafil® M2125CS), caprylocaproyl macrogolglycerides (Labrasol®), cetyl alcohol (and) ceteth-20 (and) steareth-20 (Emulcire™ 61 WL 2659), glyceryl stearate (and) PEG-75 stearate (Gelot® 64), or any mixtures thereof.

In an aspect, the emulsifying agents of the present application may act as skin penetration enhancers.

In an aspect, the composition further comprises one or more antioxidant, preservative, humectant, or plasticizer.

Antioxidants are substances which inhibit oxidation or suppress reactions promoted by oxygen or peroxides. Antioxidants, especially lipid-soluble antioxidants, can be absorbed into the cellular membrane to neutralize oxygen radicals and thereby protect the membrane. Suitable antioxdants for compositions of the present application include, but are not limited to, ascorbic acid (vitamin C), glutathione, lipoic acid, uric acid, carotenes, α-tocopherol (vitamin E), ubiquinol, butylated hydroxyanisole, butylated hydroxytoluene, sodium benzoate, propyl gallate (PG, E310), and tertiary-butylhydroquinone. The amounts of antioxidant may be from about 0.01% to about 20%, of the total weight of the composition.

Some of the excipient substances described above can have more than one function in a formulation. For example, a substance can be both a solvent and a penetration enhancer, or both a solvent and a carrier. The categorizations of materials described above are not to be construed as limiting or restricting in any manner.

The compositions can be applied directly onto affected areas of the skin, such as psoriatic plaques or dermatoses. Sprayable compositions, upon being sprayed, form droplets/mist on the affected areas and, in embodiments, can provide release of the active agent for an extended duration of time.

Addition of fatty alcohol may lead the sprayable composition may build up more viscosity however an aqueous based topical spray composition of the present application is low viscos and sprayable composition and an aqueous based topical spray composition of the present application comprises at least one fatty alcohols in the range of about 5%w/w based on total weight of the composition. Viscosities of aqueous-based emulsions of the present application frequently vary in the range of about 0.01-15 Pascal second, "Pa·s" (10-15,000 centipoise, "cP"), about 0.1-1.5 Pa·s (100-1,500 cP), or about 0.2-1 Pa·s (200-1,000 cP). In an aspect, the topical spray composition of the present application having pourable liquid like consistency and viscosity from about 100 cP to about 1000 cP when measured by Brookfield viscometer DVII+Pro, spindle LV3 at 100 rpm.

The topical spray compositions of the present application may comprise:
a) at least one betamethasone compound;
b) at least one fatty alcohol selected from group comprising of elaidyl alcohol, linoleyl alcohol, linolenyl alcohol, caproic alcohol, lauryl alcohol, stearyl alcohol, cetostearyl alcohol, behenyl alcohol, oleyl alcohol, 2-heptyl-1-undecanol, 1,17-hepatadecanediol, and mixtures thereof; c) at least one emulsifying agent; d) at least one pharmaceutically and/or dermatologically acceptable excipient; and e) water; wherein said the topical spray compositions of the present application provides more retention of betamethasone compound in skin layers and less systemic exposure of betamethasone compound.

In another aspect, the topical spray composition of the present application provides output of from about 50 mg to about 230 mg per actuation or provides output of from about 160 mg to about 190 mg per actuation.

In another aspect, the topical spray composition of the present application provides retention of betamethasones in skin layers from about 0.1% to about 20% of applied dose or about 0.1% to about 10% of applied dose.

In another aspect, the topical spray composition of the present application provides systemic exposure of betamethasones less than about 10% of applied dose or less than about 5% of applied dose.

In another aspect, penetration enhancers used in topical composition provides higher skin layer retention and lower systemic exposure by avoiding the drug entering into systemic circulation, this tendency of the penetration enhancers provides skin depot compositions.

In another aspect, oleyl alcohol is used as penetration enhancer in topical spray compositions of present application provides maximum skin retention ratio. The skin retention ratio is calculated using the formula of:

SKIN RETENTION RATIO = TOTAL BETAMETHASONES IN SKIN LAYERS/TOTAL BETAMETHASONES IN RECEPTOR.

In another aspect, the closure used for packaging are made of a polymeric substance such as high-density polyethylene (HDPE), low-density polyethylene (LDPE), or resins. The closures are particularly in the form of caps that are fitted onto the containers to aid in providing support to the dispenser unit and/or to shield the contents of the container from the outside environment. Various container materials include, but are not limited to, tin plated steel, aluminum, stainless steel, plastics, and glass.

An example of a dispenser is a unit containing a pump that can be adapted to fit on any type of container, such as by threads that match threading on the container or a self-locking joint whose mating parts exert a cam action, flexing until one part slips past a raised lip on the other part, preventing their separation. The pump is capable of dispensing sprayable compositions of the present application through a dip tube extending into a container from an actuator and attached to a one-way valve, which releases the composition from an orifice in the actuator in the form of a spray. The valve may be a metering valve.

Various types of valves that can be used include, but are not limited to, continuous spray valves and metering valves. The actuators allow for easy opening and closing of the valve and are an integral part of a package. This also serves to aid in producing the required type of product discharge. Various types of actuators include but are not limited to spray actuators, foam actuators, solid-stream actuators, and special actuators.

In another aspect, a dispensing device may be a device comprising a container, having therein a pouch system or bag containing the product, optionally fitted with a dip tube and an actuator fitted with a valve wherein the container is filled with nitrogen gas or compressed air, surrounding the pouch or bag. Containers can be made of aluminum or tin plate and the pouch system or bag containing the product can be made of layers of polyethylene (PE), polypropylene (PP), polyethylene terephthalate (PET), and aluminum. Introduction of the composition into the system further increases the pressure of the system which is capable of dispensing the composition from the pouch into the actuator, fitted with a valve, such that the composition is consistently released in the form of a substantially uniform spray upon actuation. The pouch can have a dip tube therein, communicating with the actuator valve, to control the spray rate and reduce droplet size.

In another aspect, a dispensing device useful for dispensing the compositions of the present application provides spray rates and spray patterns, in a manner such that substantially uniform dosage is dispensed each time which appreciably covers the desired affected area of the skin onto which the composition is sprayed. The pump is intended to deliver the composition uniformly onto the skin. It covers a desired area of the skin and produces very fine uniform droplets, at a specified spray rate such as, but not limited to, about 20 to about 500 mg/actuation, or about 100 to about 200 mg/actuation. The device provides a reproducible spray pattern, such as circular, frequently covering an area of about 0.1 to about 10 cm² depending on the distance from the application site.

About 2-6 priming actuations may be required for a new pump to reproducibly dispense the compositions. In a specific embodiment, about 160 µ L of a formulation is dispensed, per actuation of the pump. Devices frequently provide a reproducible distribution of droplets, in distributions where about 90% of the droplets have sizes ranging from about 1 to about 500 pm. The orifice is sized to control the droplet sizes of the dispensed product. The orifice size also affects providing of a uniform characteristic spray pattern.

In another aspect, the composition useful in treating psoriasis may be packaged in a bottle fitted with an attached spray pump closure that can be mechanically actuated by a subject or caregiver, to apply the composition to the affected skin (i.e., a pump-type spray closure).

In another aspect, a spray composition of the present application can be applied in an essentially easier and more exact way than creams and ointments can be applied. Using a spray application it is only necessary to spray a given volume, whereas the application of the semi- solid products (such as creams) requires an easily accessible and visual estimation of the cream amount or the ointment amount. Further, smearing and soiling of clothing can more easily be avoided on large surface areas using the spray compositions of the invention. For the spray compositions, spreading and rubbing are not necessary, contrary to cream and ointment products, since the layer formed on the body surface by evaporation or vaporization of the liquid already has an ideal fine dispersion of active agent; hence 'pressure pain' will not occur from the topical application of spray compositions of the present application.

In an aspect, queous based emulsion sprayable compositions of the present application also permit applying a medicament by a method whereby the area of application is contacted by only the spray (i.e.,) elbows, knees, scalp, and back. An aqueous based emulsion sprayable composition of the present application is self-administered to area of application is contacted by only the spray (i.e.,) elbows, knees, scalp, and back.

In an aspect, the claimed composition is for use in a method of treating atopic dermatitis, seborrhoeic dermatitis, eczema, and psoriasis, said method comprising administering a pharmaceutically and/or dermatologically effective amount of topical spray composition directly onto an affected part of the skin of a subject in need thereof.

In an aspect, the claimed composition for use in treating atopic dermatitis, seborrhoeic dermatitis, eczema and psoriasis is applied comprising steps of: providing a device having a topical spray composition comprising: a betamethasone compound; and delivering a spray of said composition directly onto an affected part of the skin of a subject in need thereof, wherein the said method provides spray characteristics of a wide angle full cone spray pattern having the first axis of from about 35 mm to about 60 mm, the second axis of from about 35 mm to about 55 mm, and the ratio between of first and second axis is from about 1 to about 1.5.

In an aspect, the administration distance is from about 20 mm to about 60 mm from subject's skin to device and the spray angle is from about 50 to about 70 degrees to the subject's skin.

In an aspect, the claimed composition may be used in a method of administering the topical spray composition comprising steps of: providing a device having a topical spray composition comprising: a corticosteroid; and delivering a spray of said composition directly onto an affected part of the skin of a subject in need thereof, wherein the said device delivers from about 65 mg to about 210 mg of spray composition per stroke, wherein the spray count from about 230 to about 270 strokes to empty the composition in the device.

In an aspect, topical application of compositions of the present application forms a depot on the skin without forming an occlusive film, thereby extending the duration of active agent action while allowing 'breathing' of the skin.

Another aspect of the present application, which is not claimed, further provides processes for preparing compositions that can be filled into suitable dispensing devices. In embodiments, processes comprise:
a) preparing a composition comprising the active agent and one or more suitable excipients, and
b) filling a desired quantity of the composition into a dispensing device.

In another aspect, which is not claimed, processes for preparing topical compositions comprising betamethasone as an active agent and excipients comprise:
a) heating a mixture comprising an emulsifying agent and a solvent to obtain an oily phase;
b) optionally, admixing an antioxidant and/or preservative into the oily phase of a);
c) admixing a corticosteroid with a penetration enhancer;
d) admixing the material of c) with material of a) or b);
e) dissolving a polymer in an aqueous phase;
f) admixing the oily phase of d) slowly with an aqueous phase of e) with continuous mixing; and
g) homogenizing the mixture of f), followed by cooling.

In another aspect, compositions of the present application have pH values ranging from about 3 to about 7, or from about 3.5 to about 6.

In another aspect, the oily phase for an emulsion is a mixture of emulsifying agents and a solvent.

In another aspect, betamethasone spray compositions of the present application exhibit a comparable drug dissolution profile to that of a commercial DIPROLENE Lotion Augmented, 0.05%(containing 0.05% betamethasone base), water, isopropyl alcohol (30%), hydroxypropyl cellulose, propylene glycol, sodium phosphate, phosphoric acid, and sodium hydroxide.

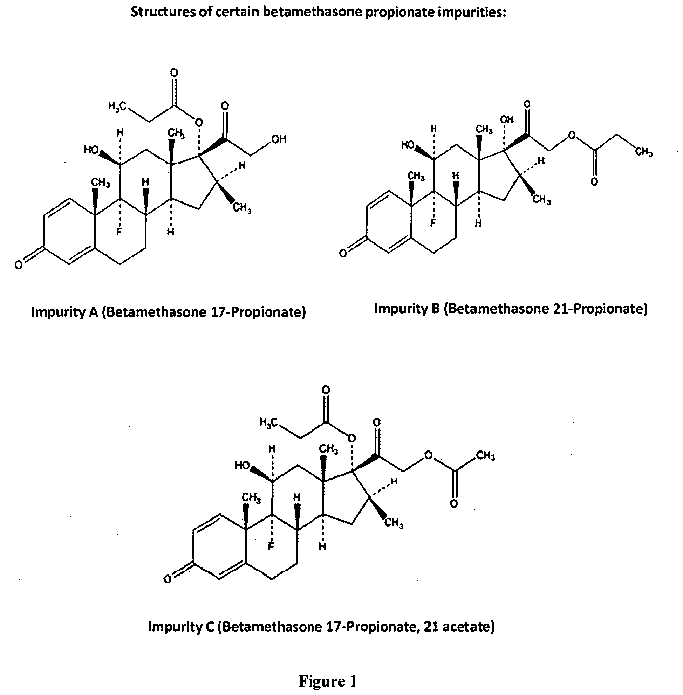

In another aspect, betamethasone propionate compositions of the present application may contain any one or more of impurities, such as impurity A (betamethasone 17-propionate) in amounts not more than about 5%, impurity B (betamethasone 21-propionate) in amounts not more than about 2%, impurity C (betamethasone 17-propionate 21-acetate) in amounts not more than about 1%, and single unknown impurity in amounts not more than about 1.0% (these impurities have the structures shown in Figure 1), and any other drug-related impurities, in amounts such that any such impurities do not substantially adversely affect the safety of the composition. Impurities A and B are primarily observed during stability studies of a formulation, and impurity C is generally a process-related impurity from synthesis of the drug. The above impurity limits are expressed as percentages of the label drug content in the composition.

In another aspect, betamethasone dipropionate compositions of the present application may comprise one or more unknown impurities. One of such an impurity of the betamethasone dipropionate is enol aldehyde impurity (Impurity D). Enol aldehydes are known to be degradation products of corticosteroids having 1, 3-dihydroxyacetone side chain on their D-ring, such as betamethasone, dexamethasone, beclomethasone and the like. Enol aldehyde impurities formed from these corticosteroids via acid-catalysed beta elimination of water from side chain and enol aldehydes could also be formed from the corresponding 17, 21-diesters of these corticosteroids in alkaline conditions. It has been found that enol aldehyde formation or degradation is increased with increase of the temperature.

### E-isomer of bethamethasone enol aldehyde

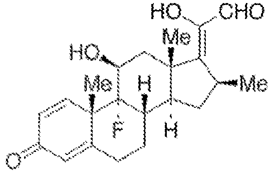

Various conditions such as pH of the composition, and storing conditions influences the formation of enol aldehyde and the enol aldehyde is known to exist in two different isomers E-isomer and Z-isomer. However, the ratio between E and Z isomers may be different depending on the conditions such as pH of the formulation, medium, and temperature. It has been found that E-isomer formation is increased by increase in temperature.

In another aspect, betamethasone propionate compositions of the present application may comprise Impurity D in the amounts of from about 0.001% w/w to about 1.3% w/w of the label drug content.

Surprisingly, in one aspect of the application, the enol aldehyde impurity is controlled well below 1% for period of at least 6 months at 25°C, or for a period of at least 12 months at 25°C, or for a period of at least 18 months at 25°C, or for a period of at least 24 months at 25°C.

The following examples are provided to illustrate certain specific aspects and embodiments of the application, and are not to be construed as limiting the scope of the application in any manner.

### EXAMPLES

In the examples, the active agent betamethasone dipropionate used had a particle size distribution wherein half of the particles had sizes less than about 50 pm, and 90% of the particles had sizes less than about 300 µm. Example 6 is according to the invention.

### EXAMPLES: Betamethasone spray compositions:

| **Examples** | **Example 1** | **Example 2** | **Example 3** | **Example 4** | **Example 5** | **Example 6** |
|---|---|---|---|---|---|---|
| **Ingredients** | **w/w** | **w/w** | **w/w** | **w/w** | **w/w** | **w/w** |
| Betamethasone dipropionate | 0.0643 | 0.0643 | 0.0643 | 0.0643 | 0.0643 | 0.0643 |
| Sorbitan monostearate | 4.58 | 4.58 | 4.58 | 4.58 | 4.58 | 4.58 |
| Polyoxyl 20 Cetostearyl Ether | 2.42 | 2.42 | 2.42 | 2.42 | 2.42 | 2.42 |
| Cetostearyl alcohol | 1 | 1 | 1 | 1 | 1 | 1 |
| Mineral Oil | 7.06 | 7.06 | 7.06 | 7.06 | 7.06 | 7.06 |
| Oleyl Alcohol | - | - | - | - | - | 5 |
| Elaidyl alcohol | 5 | - | - | - | - | - |
| Caproic alcohol | - | 5 | - | - | - | - |
| Lauryl alcohol | - | - | 5 | - | - | - |
| Stearyl alcohol | - | - | - | 5 | - | - |
| Behenyl alcohol | - | - | - | - | 5 | - |
| Propyl paraben | 0.8 | 0.8 | 0.8 | 0.8 | 0.8 | 0.8 |
| Methyl paraben | 0.2 | 0.2 | 0.2 | 0.2 | 0.2 | 0.2 |
| Butylated hydroxy toluene | 0.05 | 0.05 | 0.05 | 0.05 | 0.05 | 0.05 |
| Hydroxyethyl cellulose | 0.05 | 0.05 | 0.05 | 0.05 | 0.05 | 0.05 |
| Purified water | 78.7757 | 78.7757 | 78.7757 | 78.7757 | 78.7757 | 78.7757 |

Manufacturing process:
i. Drug Solution Preparation: Betamethasone dipropionate and butylated hydroxyl toluene were solubilized in oleyl alcohol with constant stirring;
ii. Oil Phase preparation: Sorbitan monostearate, polyoxyl 20 cetostearyl ether, cetostearyl alcohol and mineral oil were heated in a stainless steel container up to 70 ± 2° C. Propyl paraben was added to the oil phase;
iii. Drug solution prepared in step 1 was slowly added to oil phase under stirring. Temperature of the stainless steel vessel under 70 ± 2° C;
iv. Aqueous phase preparation: water and methyl paraben was homogenized and added a quantity of hydroxyl ethyl cellulose to prepare aqueous phase;
v. Oil phase and aqueous phase were homogenized. Homogenization was continued for 10 minutes at 2400 rpm and
vi. Then, the vessel was cooled at 30° C ± 2° C under stirring at 250 rpm using water jacket and allowed to cool to ambient temperature.

### Stability testing of Example 6

The prepared formulations, filled into closed containers, were exposed to the stability testing conditions: 25°C and 60% relative humidity (RH), 30°C and 65% RH, and 40°C and 75% RH for two months. All samples remained off-white homogenous emulsions with no phase separation. Drug assay values are within the specified limits of 90-110% of the label drug Content. Studies at various storage points are shown in Table 1, where the values are percentages of the label drug content.

**Stability Study Table 1**

| **Storage Conditions** | | **Drug Assay** | **Impurities** | | | | |
|---|---|---|---|---|---|---|---|
| | | | **A** | **B** | **C** | **D** | **Total** |
| Initial | -- | 100.2 | 0.14 | 0.03 | ND | ND | 0.17 |
| 15 days | 2-8°C | 100.1 | ND | ND | ND | ND | 0.00 |
| | 25°C | 101.4 | ND | 0.02 | ND | ND | 0.02 |
| | 30°C | 99.6 | 0.15 | 0.06 | ND | ND | 0.21 |
| | 40°C | 100.1 | 0.07 | 0.03 | ND | ND | 0.10 |
| 1 Months | 2-8°C | 101.3 | 0.03 | ND | ND | ND | 0.03 |
| | 25°C | 101.2 | 0.06 | 0.05 | ND | ND | 0.11 |
| | 30°C | 100.9 | 0.09 | 0.05 | ND | ND | 0.14 |
| | 40°C | 100.4 | 0.20 | 0.15 | ND | 0.02 | 0.59 |
| 2 Months | 2-8°C | 102.8 | 0.04 | 0.05 | ND | ND | 0.09 |
| | 25°C | 103.5 | 0.10 | 0.07 | ND | ND | 0.17 |
| | 30°C | 103.3 | 0.11 | 0.08 | ND | ND | 0.18 |
| | 40°C | 102.2 | 0.32 | 0.38 | ND | 0.44 | 1.13 |
| 3 Months | 2-8°C | 99.5 | ND | 0.08 | ND | ND | 0.08 |
| | 25°C | 100.0 | 0.13 | 0.06 | ND | 0.07 | 0.27 |
| | 30°C | 100.1 | 0.20 | 0.13 | ND | 0.15 | 0.49 |
| | 40°C | 98.0 | 0.38 | 0.56 | ND | 0.68 | 1.62 |
| 6 Months | 2-8°C | 101.2 | 0.09 | ND | ND | ND | 0.09 |
| | 25°C | 102.2 | 0.21 | 0.15 | ND | 0.15 | 0.51 |
| | 30°C | 100.0 | 0.35 | 0.31 | ND | 0.3 | 0.97 |
| | 40°C | 98.8 | 0.67 | 1.09 | ND | 1.12 | 2.98 |
| 9 Months | 2-8°C | 100.7 | 0.11 | ND | ND | ND | 0.11 |
| | 25°C | 102.5 | 0.23 | 0.20 | ND | 0.17 | 0.60 |
| | 30°C | 100.9 | 0.39 | 0.47 | ND | 0.44 | 1.30 |
| 12 Months | 2-8°C | 102.5 | 0.16 | 0.12 | ND | ND | 0.28 |
| | 25°C | 96.2 | 0.30 | 0.26 | ND | 0.12 | 0.68 |
| | 30°C | 98.4 | 0.46 | 0.55 | ND | 0.37 | 1.38 |
| 18 Months | 2-8°C | 101.2 | 0.18 | 0.15 | ND | 0.14 | 0.47 |
| | 25°C | 103.1 | 0.36 | 0.41 | ND | 0.31 | 1.08 |
| 24 Months | 2-8°C | 100.2 | 0.27 | 0.20 | ND | 0.13 | 0.60 |
| | 25°C | 101.7 | 0.43 | 0.51 | ND | 0.39 | 1.33 |

| | | | | | | | |
|---|---|---|---|---|---|---|---|
| ND = not detected; | | | | | | | |

| **Examples** | **Details of solution compositions of betamethasone dipropionate** |
|---|---|
| **Example 7** | Solution of betamethasone dipropionate (0.0643%w/w) in Ethanol + Propylene Glycol (1:1)+Eladiyl alcohol (5%w/w) |
| **Example 8** | Solution of betamethasone dipropionate (0.0643%w/w) in Ethanol + Propylene Glycol (1:1)+ Caproic alcohol (5%w/w) |
| **Example 9** | Solution of betamethasone dipropionate ( 0.0643%w/w) in Ethanol + Propylene Glycol (1:1)+Lauryl alcohol (5%w/w) |
| **Example 10** | Solution of betamethasone dipropionate (0.0643%w/w) in Ethanol + Propylene Glycol (1:1)+ Stearyl alcohol (5%w/w) |
| **Example 11** | Solution of betamethasone dipropionate (0.0643%w/w) in Ethanol + Propylene Glycol (1:1)+ Behenyl alcohol (5%w/w) |
| **Example 12** | Solution of betamethasone dipropionate (0.0643%w/w) in Ethanol + Propylene Glycol (1:1)+Oleyl alcohol(5%w/w) |
| **Example 13** | Solution of betamethasone dipropionate (0.0643%w/w) in Ethanol + Propylene Glycol (1:1)+Menthol (5%w/w) |
| **Example 14** | Solution of betamethasone dipropionate (0.0643%w/w) in Ethanol + Propylene Glycol (1:1)+Alpha Terpeniol (5%w/w) |
| **Example 15** | Solution of betamethasone dipropionate (0.0643%w/w) in Ethanol + Propylene Glycol (1:1)+Transcutol (5%w/w) |
| **Example 16** | Solution of betamethasone dipropionate (0.0643%w/w) in Ethanol + Propylene Glycol (1:1)+Isopropyl Myristate (5%w/w) |

### Example 17: Topical Absorption and penetration study of Examples 1-16 compositions Topical spray compositions of the present applications were screened for the penetration of drug into different layers of skin and permeation into the receptor phase by finite dosing method using vertical diffusion cells (Franz-type)

### Methods and Materials:

There were sixteen treatment groups (n=9 cells for each). Each group was having 3 skin samples received from 3 different donors (55 years old or younger; 3 donors X 3 replicates). All the test compositions were stored at room temperature.

Skin model: Human cadaver skin was used in this study. The dermatomed human cadaver skin tissue with average thickness of about 350-450 pm. The donor tissue was divided evenly among the diffusion cells.

*In vitro* percutaneous absorption and penetration study: the topical spray compositions of Examples 1-16 were screened using vertical diffusion cells (Franz-type). The skin samples were mounted on individual diffusion cells. Each cell in the station was having diffusion area of 0.503 cm² (8mm in diameter). Each individual cell was static Franz-cell type. The receptor chamber was filled with 3.0 ml of 4% BSA in water supplemented with 0.01% gentamicin sulfate, which will be vigorously and continually mixed. The temperature was set at 32±0.2°C. The cell was incubated at 32°C for one hour before dosing. At end of incubation period, samples from the receptor fluid were taken as the t=0 samples. A fresh batch of receptor fluid pre-incubated at 32°C was introduced into the receptor chamber in the HTS cells. The compositions were dosed at a level of approximately 2.5 mg per cell, which was equivalent to 5mg/cm² and they were applied using a positive displacement pipette. The dosing volumes were calculated by calculating density of each composition. The samples were collected at the time intervals of 0 hour, 2 hours, 6 hours, 10 hours, 12 hours or 24 hours and stored in a freezer before analysis. Skin penetration was analyzed by samples collected at 0, 2, 6, 10, 12 or 24 hours and the full mass balance study was conducted after 24 hours.

The full mass balance study amount of betamethasone dipropionate and its metabolites were analysed from following skin locations: skin surface (unabsorbed, and/or unpenetrated), stratum corneum (from tape stripping), and epidermis (separation from dermis and followed by solvent extraction), dermis (separation from epidermis and followed by solvent extraction). The tissue surface was wiped with Q-tip wetted with 1X PBS three times to remove unabsorbed and unpenetrated API (i.e.,) betamethasone and its metabolites. The standard tape-stripping method was used to remove the stratum corneum (SC) layer. After removal of stratum corneum layer, the remaining tissue was wetted with 1X PBS, epidermis and dermis layers were separated mechanically.

All collected samples were analyzed using LC-MS/MS with qualified method for following analytes: betamethasone dipropionate, betamethasone 17-propionate, betamethasone 21-propionate and betamethasone base. Betamethasone 17-propionate, betamethasone 21-propionate and betamethasone base are the metabolites of the parent drug betamethasone dipropionate.

**Table 2: Betamethasones retention in skin layers**

| **Examples** | **Stratum Corneum (ng)** | **Epidermis (ng)** | **Dermis (ng)** | **Total (ng)** |
|---|---|---|---|---|
| Example 1 | 5.40 | 17.09 | 14.22 | 36.71 |
| Example 2 | 11.11 | 15.23 | 4.87 | 31.21 |
| Example 3 | 36.83 | 42.55 | 33.81 | 113.19 |
| Example 4 | 2.02 | 1.89 | 0.79 | 4.70 |
| Example 5 | 17.57 | 7.43 | 6.38 | 31.39 |
| Example 6 | 33.00 | 18.34 | 14.11 | 65.45 |
| Example 7 | 82.65 | 198.11 | 40.52 | 321.28 |
| Example 8 | 78.76 | 244.14 | 57.68 | 380.59 |
| Example 9 | 78.61 | 162.92 | 67.49 | 309.01 |
| Example 10 | 70.48 | 199.21 | 49.78 | 319.47 |
| Example 11 | 76.09 | 182.91 | 86.33 | 345.32 |
| Example 12 | 73.29 | 206.15 | 54.89 | 334.32 |
| Example 13 | 108.23 | 197.96 | 53.02 | 359.21 |
| Example 14 | 141.82 | 165.20 | 68.15 | 375.17 |
| Example 15 | 184.39 | 133.52 | 106.10 | 424.01 |
| Example 16 | 236.14 | 98.17 | 87.07 | 421.39 |

**Table 3: Percentage of betamethasone retention in skin layers and Receptor level**

| **Examples** | **Applied dose (in ng)** | **Skin retention dose (in ng)** | **Receptor level after 24 Hours (in ng)** | **Percentage of retention in skin layers** | **Percentage permeated into receptor** |
|---|---|---|---|---|---|
| Example 1 | 1500 | 36.71 | 11.26 | 2.45 | 0.75 |
| Example 2 | 1360 | 31.21 | 19.35 | 2.29 | 1.42 |
| Example 3 | 1520 | 113.19 | 50.69 | 7.45 | 3.33 |
| Example 4 | 745 | 4.70 | 2.31 | 0.63 | 0.31 |
| Example 5 | 1370 | 31.39 | 2.78 | 2.29 | 0.20 |
| Example 6 | 1255 | 65.45 | 11.82 | 5.22 | 0.94 |
| Example 7 | 1340 | 321.28 | 188.78 | 23.98 | 14.09 |
| Example 8 | 1385 | 380.59 | 124.92 | 27.48 | 9.02 |
| Example 9 | 1340 | 309.01 | 130.58 | 23.06 | 9.74 |
| Example 10 | 1520 | 319.47 | 132.89 | 21.02 | 8.74 |
| Example 11 | 1445 | 345.32 | 241.54 | 23.90 | 16.72 |
| Example 12 | 1190 | 334.32 | 16.64 | 28.09 | 1.40 |
| Example 13 | 1165 | 359.21 | 50.69 | 30.83 | 4.35 |
| Example 14 | 1160 | 375.17 | 183.24 | 32.34 | 15.80 |
| Example 15 | 1055 | 424.01 | 237.29 | 40.19 | 22.49 |
| Example 16 | 1300 | 421.39 | 211.86 | 32.41 | 16.30 |

**Table 4: Skin retention ratio range (n=9 cells)**

| **Examples** | Minimum | Maximum |
|---|---|---|
| Example 1 | 1.4 | 8.4 |
| Example 2 | 0.7 | 8.3 |
| Example 3 | 1.1 | 13.7 |
| Example 4 | 0.9 | 7.3 |
| Example 5 | 0.7 | 49.6 |
| Example 6 | 1.5 | 47.7 |
| Example 7 | 0.2 | 10.5 |
| Example 8 | 1.3 | 10.4 |
| Example 9 | 0.9 | 10.0 |
| Example 10 | 0.6 | 24.1 |
| Example 11 | 0.4 | 3.9 |
| Example 12 | 5.7 | 82.1 |
| Example 13 | 2.8 | 81.5 |
| Example 14 | 0.7 | 3.8 |
| Example 15 | 0.3 | 10.1 |
| Example 16 | 1.1 | 5.0 |

### Example 18: Irritation Patch Test Study of betamethasone dipropionate spray

Total forty (40) subjects were enrolled and out of which thirty four (34) had completed the study. This was a randomized, double-blind, single-center, vehicle-controlled, within-subject comparison patch test study of followings for irritation potential when repeatedly applied to the skin under semi-occlusive conditions in healthy volunteers:
i. Betamethasone dipropionate Spray (Example 6),
ii. Vehicle spray (without active agent of example 6),
iii. Vehicle lotion (isopropyl alcohol, hydroxypropyl cellulose, sodium phosphate monobasic monohydrate, propylene glycol, phosphoric acid, sodium hydroxide and water i.e. vehicle for DIPROLENE Lotion Augmented 0.05%,
iv. Sodium lauryl sulfate (SLS) 0.2% and
v. Saline 0.9%

All subjects received applications of each study product to intact skin at randomly assigned, adjacent sites on the back. Evaluators and subjects were blinded and unaware of the identity of the study product at the patch test sites. The study products were applied under semi-occlusive patch conditions using a 2 cm x 2 cm patch. The products were applied to either side of the infrascapular area of the back. Evaluation of dermal reactions at the application sites were assessed clinically using a visual scale that rates the degree of erythema, edema, and other signs of cutaneous irritation.

A total of 21 patch applications were made over a period of 21 days. Irritancy score of each compositions were recorded.

Conclusion: Significantly more irritation was observed at the Vehicle Lotion site and 0.2% SLS site than at the betamethasone dipropionate spray site, vehicle spray site and 0.9% sterile saline site. There was no significant difference in irritation between the vehicle lotion site and 0.2% SLS site, and no significant difference in irritation between the betamethasone dipropionate spray site and vehicle spray site, the betameth0.9% sterile saline site. Under the exaggerated conditions of use in this study, with continuous exposure under semi occlusion for 21 days, betamethasone dipropionate spray (Example 6) and its vehicle spray (Example 6 without active agent) produced no evidence of significant irritation. In comparison, the vehicle lotion (vehicle for DIPROLINE Lotion augmented 0.05%) produced mild irritation, as there was no significant difference in irritation between the Vehicle Lotion site and 0.2% SLS site, a known mild irritant.

### Example 19: Spray Characteristics of Example 6 composition:

The spray pattern characterizes the spray following impaction on an appropriate target (i.e.,) a thin layer chromatography (TLC) plate. A TLC plate having silica gel 60, F254 (florescence indicator), 250 µm thick layer on glass was used as target in present study and the TLC plate was held with suitable fastener. Automatic air pressure actuation device were used in the study to automate the spray actuations. Mark VII® Max pumps (1-10) were used to pump the composition in the spray pattern studies. The spray distance was 40mm from the spray nozzle to the TLC plate. The sprayer (the container is a2oz HDPE bottle) was loaded with compositions of Example 1 and the composition density was 0.9081 g/ml and Kern ALJ220-4NM was used to measure the output from each stroke. Compositions were shaken three times before priming and priming the pump 10X into a hood was done to ensure a full stroke. Sprayer and TLC plate with fastener were brought into right position at 40mm distance. Actuation profile was chosen as the pump output is 0.16ml; actuation/return Velocity was 100 mm/s; actuation/return acceleration was 5700 mm/s2; initial delay was 0 ms; hold time was 100 ms; final delay was 0 ms and inter actuation delay was 0 ms. After spray, the TLC plate was taken away from the fastener and the spray pattern was viewed under 254 nm UV light and a suitable camera was used to take pictures (eg. Digital camera) in ultraviolet light and minimum and maximum diameters of spray patterns were determined. This test was repeated for 28 days (2 times a day) and compositions were stored in room temperature horizontally and upright positions between the daily tests.

## Claims

1. An aqueous topical spray composition comprising:
a) a corticosteroid which is a betamethasone compound, such as betamethasone benzoate, betamethasone dipropionate, betamethasone sodium phosphate, betamethasone valerate and combinations thereof;
b) a fatty alcohol acting as a skin penetration enhancer, wherein the fatty alcohol comprises oleyl alcohol and is present in an amount of about 0.001% to about 15% by weight based on the total weight of the composition;
c) at least one pharmaceutically and/or dermatologically acceptable excipient and
d) water; and
e) wherein the composition is free of propylene glycol, free of propellant and substantially non-foaming, such that the topical spray composition forms mist or droplet in more than 90% of quantity when sprayed onto the affected skin area.

2. The composition of claim 1, wherein said composition does not form any film layer.

3. The composition of claim 1, wherein the skin penetration enhancer further comprises at least one other fatty alcohol selected from the group comprising of elaidyl alcohol, linoleyl alcohol, linolenyl alcohol, caproic alcohol, lauryl alcohol, stearyl alcohol, behenyl alcohol, cetostearyl alcohol, 2-heptyl-1-undecanol, : 1, 17-heptadecanediol, and combinations thereof.

4. The composition of claim 1, wherein said betamethasone compound is selected from the group consisting of betamethasone benzoate, betamethasone dipropionate, betamethasone sodium phosphate, betamethasone valerate, and combinations thereof and said betamethasone compound is optionally present in amounts equivalent to 0.025 percent to 0.1 percent by weight of product.

5. The composition of claim 1, wherein said pharmaceutically and/or dermatologically acceptable excipient is selected from the group consisting of an emulsifying agent, a polymer, and combination thereof:
wherein the emulsifying agent is selected from the group consisting of a cationic surfactant, an anionic surfactant, a zwitterionic surfactant, an amphoteric surfactant, Polyoxyl 20 Cetostearyl Ether, and combinations thereof; and
wherein the polymer is selected from the group consisting of a carbomer, polyethylene glycol, acrylate polymer, methacrylate polymer, polyvinylpyrrolidone, copolymer based on butyl methacrylate and methyl methacrylate, vinyl acetate, polyvinyl acetate, cellulose, gum, alginate, cellulose acetate phthalate, cellulose acetate butyrate, hydroxypropyl methylcellulose phthalate, and combinations thereof.

6. The composition of claim 1, further comprising one or more excipients selected from the group consisting of antioxidant, preservative, humectant, and plasticizer.

7. The composition of claim 1, wherein said composition is substantially nonirritating to the subject's skin.

8. The composition of claim 1, wherein said composition is stable at least for the period of about 6 months at 40°C or at least for the period of 24 months at 25°C.

9. The composition of claim 1, wherein said betamethasone compound is betamethasone dipropionate.

10. The composition of claim 1, wherein enol aldehyde impurity in the said composition is below about 1% for period of at least 12 months at 25°C; wherein enol aldehyde impurity in the said composition is below about 1% for period of at least 18 months at 25°C; wherein enol aldehyde impurity in the said composition is below about 1% for period of at least 24 months at 25°C.

11. The composition of claim 1, wherein said composition provides retention of betamethasones in skin layers from 0.1% to 10% of applied dose.

12. The composition of claim 1, wherein said composition is a skin depot composition.

13. The composition of claim 1, wherein said composition is an emulsion selected from the group consisting of an aqueous based emulsion and an oil-in-water emulsion.

14. A topical spray composition for use in the treatment of atopic dermatitis, seborrhoeic dermatitis, eczema and psoriasis, wherein the topical spray composition is to be administered by the steps of: providing a device having the topical spray composition of claim 1; and administering spray of said composition directly onto an affected part of the skin of a subject in need thereof.

15. The topical spray composition for use according to claim 14, wherein said method provides spray characteristics of a wide angle full cone spray pattern having the first axis of from 35 mm to 60 mm, the second axis of from 35 mm to 55 mm, and a ratio between the first and second axis that is from 1 to 1.5.

16. The topical spray composition for use according to claim 15, wherein the method delivers from 65 mg to 210 mg of composition per stroke, wherein the spray count from about 230 to about 270 strokes to empty the composition in the device.

17. The topical spray composition for use according to claim 14, wherein the administration distance is from 20 mm to 60 mm from subject's skin to device and the spray angle is from 50 to 70 degrees to the subject's skin.

18. The topical spray composition for use according to claim 14, wherein said composition forms a depot on the skin without forming an occlusive film; or wherein said composition is an aqueous based emulsion.

19. The topical spray composition for use according to claim 14, wherein the psoriasis is moderate to severe plaque psoriasis.

## Patentansprüche

1. Wässrige topische Sprühzusammensetzung, umfassend:
a) ein Kortikosteroid, das eine Betamethasonverbindung ist, wie beispielsweise Betamethasonbenzoat, Betamethasondipropionat, Betamethasonnatriumphosphat, Betamethasonvalerat und Kombinationen davon,
b) einen Fettalkohol, der als ein Hautpenetrationsverstärker wirkt, wobei der Fettalkohol Oleylalkohol umfasst und in einer Menge von etwa 0,001 bis etwa 15 Gew.-%, bezogen auf das Gesamtgewicht der Zusammensetzung, vorhanden ist,
c) mindestens ein pharmazeutisch und/oder dermatologisch annehmbaren Exzipiens und
d) Wasser, und
e) wobei die Zusammensetzung frei von Propylenglykol, treibmittelfrei und im Wesentlichen nicht schäumend ist, sodass die topische Sprühzusammensetzung Nebel oder Tröpfchen in mehr als 90% der Menge bildet, wenn sie auf den betroffenen Hautbereich gesprüht wird.

2. Die Zusammensetzung nach Anspruch 1, wobei die Zusammensetzung keine Filmschicht bildet.

3. Zusammensetzung nach Anspruch 1, wobei der Hautpenetrationsverstärker ferner mindestens einen anderen Fettalkohol umfasst, der aus der Gruppe ausgewählt ist, die Elaidylalkohol, Linoleylalkohol, Linolenylalkohol, Caprinalkohol, Laurylalkohol, Stearylalkohol, Behenylalkohol, Cetostearylalkohol, 2-Heptyl-1-undecanol, 1,17-Heptadecandiol und Kombinationen davon umfasst,

4. Zusammensetzung nach Anspruch 1, wobei die Betamethasonverbindung aus der Gruppe ausgewählt ist, die aus Betamethasonbenzoat, Betamethasondipropionat, Betamethasonnatriumphosphat, Betamethasonvalerat und Kombinationen davon besteht, und die Betamethasonverbindung gegebenenfalls in Mengen vorhanden ist, die 0,025 bis 0,1 Gewichtsprozent des Produkts entsprechen.

5. Zusammensetzung nach Anspruch 1, wobei das pharmazeutisch und/oder dermatologisch annehmbare Exzipiens ausgewählt ist aus der Gruppe bestehend aus einem Emulgator, einem Polymer und einer Kombination davon,
wobei der Emulgator ausgewählt ist aus der Gruppe, bestehend aus einem kationischen Tensid, einem anionischen Tensid, einem zwitterionischen Tensid, einem amphoteren Tensid, Polyoxyl-20-cetostearylether und Kombinationen davon, und
wobei das Polymer ausgewählt ist aus der Gruppe bestehend aus einem Carbomer, Polyethylenglykol, Acrylatpolymer, Methacrylatpolymer, Polyvinylpyrrolidon, Copolymer auf der Basis von Butylmethacrylat und Methylmethacrylat, Vinylacetat, Polyvinylacetat, Cellulose, Gummi, Alginat, Celluloseacetatphthalat, Celluloseacetatbutyrat, Hydroxypropylmethylcellulosephthalat und Kombinationen davon.

6. Zusammensetzung nach Anspruch 1, ferner umfassend einen oder mehrere Hilfsstoffe, ausgewählt aus der Gruppe bestehend aus Antioxidationsmittel, Konservierungsmittel, Feuchthaltemittel und Weichmacher.

7. Zusammensetzung nach Anspruch 1, wobei die Zusammensetzung für die Haut des Patienten im Wesentlichen nicht reizend ist.

8. Zusammensetzung nach Anspruch 1, wobei die Zusammensetzung mindestens für die Dauer von etwa 6 Monaten bei 40°C oder mindestens für die Dauer von 24 Monaten bei 25°C stabil ist.

9. Zusammensetzung nach Anspruch 1, wobei es sich bei der Betamethasonverbindung um Betamethasondipropionat handelt.

10. Zusammensetzung nach Anspruch 1, wobei die Enol-Aldehyd-Verunreinigung in der Zusammensetzung über einen Zeitraum von mindestens 12 Monaten bei 25°C unter etwa 1% liegt, wobei die Enol-Aldehyd-Verunreinigung in der Zusammensetzung über einen Zeitraum von mindestens 18 Monaten bei 25°C unter etwa 1% liegt, wobei die Enol-Aldehyd-Verunreinigung in der Zusammensetzung über einen Zeitraum von mindestens 24 Monaten bei 25°C unter etwa 1% liegt.

11. Zusammensetzung nach Anspruch 1, wobei die Zusammensetzung eine Retention von Betamethasonen in Hautschichten von 0,1% bis 10% der angewandten Dosis gewährt.

12. Zusammensetzung nach Anspruch 1, wobei die Zusammensetzung eine Hautdepotzusammensetzung ist.

13. Zusammensetzung nach Anspruch 1, wobei die Zusammensetzung eine Emulsion ist, ausgewählt aus der Gruppe bestehend aus einer Emulsion auf wässriger Basis und einer Ölin-Wasser-Emulsion.

14. Topische Sprühzusammensetzung zur Verwendung bei der Behandlung von atopischer Dermatitis, seborrhoischer Dermatitis, Ekzemen und Psoriasis, wobei die topische Sprühzusammensetzung durch die folgenden Schritte zu verabreichen ist: Bereitstellen einer Vorrichtung mit der topischen Sprühzusammensetzung nach Anspruch 1 und Verabreichen von Sprühnebel dieser Zusammensetzung direkt auf einen betroffenen Teil der Haut eines Subjekts, das dies benötigt.

15. Topische Sprühzusammensetzung zur Verwendung nach Anspruch 14, wobei das Verfahren Sprühcharakteristika eines Weitwinkel-Vollkegel-Sprühmusters mit der ersten Achse von 35 mm bis 60 mm, der zweite Achse von 35 mm bis 55 mm und einem Verhältnis zwischen der ersten und zweiten Achse von 1 bis 1,5 aufweist.

16. Topische Sprühzusammensetzung zur Verwendung nach Anspruch 15, wobei das Verfahren von 65 mg bis 210 mg der Zusammensetzung pro Hub abgibt, wobei die Sprühanzahl von etwa 230 bis etwa 270 Hübe beträgt, um die Zusammensetzung in der Vorrichtung zu entleeren.

17. Topische Sprühzusammensetzung zur Verwendung nach Anspruch 14, wobei der Verabreichungsabstand von 20 mm bis 60 mm von der Haut des Patienten zur Vorrichtung und der Sprühwinkel von 50 bis 70 Grad zur Haut des Patienten beträgt.

18. Topische Sprühzusammensetzung zur Verwendung nach Anspruch 14, wobei die Zusammensetzung ein Depot auf der Haut bildet, ohne einen okklusiven Film zu bilden, oder wobei die Zusammensetzung eine Emulsion auf wässriger Basis ist.

19. Topische Sprühzusammensetzung zur Verwendung nach Anspruch 14, wobei die Psoriasis eine mittelschwere bis schwere Plaque-Psoriasis ist.

## Revendications

1. Composition aqueuse de pulvérisation topique comprenant :
a) un corticostéroïde qui est un composé de la bétaméthasone, tel que benzoate de bétaméthasone, dipropionate de bétaméthasone, phosphate sodique de bétaméthasone, valérate de bétaméthasone et des combinaisons de ceux-ci,
b) un alcool gras agissant en tant qu'activateur de pénétration cutanée, dans lequel l'alcool gras comprend de l'alcool oléylique et est présent en une quantité d'environ 0,001 % à environ 15 % en poids par rapport au poids total de la composition,
c) au moins un excipient pharmaceutiquement et/ou dermatologiquement acceptable, et
d) de l'eau, et
e) la composition étant exempte de propylène glycol, exempte de gaz propulseur et sensiblement non moussante, de sorte que la composition de pulvérisation topique forme un brouillard ou des gouttelettes en quantité supérieure à 90 % lorsqu'elle est pulvérisée sur la zone cutanée affectée.

2. Composition selon la revendication 1,
la composition ne formant pas de couche pelliculaire.

3. Composition selon la revendication 1, dans laquelle l'activateur de pénétration cutanée comprend en outre au moins un autre alcool gras choisi dans le groupe comprenant : alcool élaidylique, alcool linoléylique, alcool linolénique, alcool caproique, alcool laurylique, alcool stéarylique, alcool béhénylique, alcool cétostéarylique, 2-heptyl-1-undécanol, : 1, 17-heptadécanediol et leurs combinaisons.

4. Composition selon la revendication 1, dans laquelle le composé de bétaméthasone est choisi dans le groupe comprenant :
benzoate de bétaméthasone, dipropionate de bétaméthasone, phosphate de sodium de bétaméthasone, valérate de bétaméthasone et leurs combinaisons, et
le composé de bétaméthasone est éventuellement présent en des quantités équivalant à 0,025 % à 0,1 % en poids du produit.

5. Composition selon la revendication 1, dans laquelle l'excipient pharmaceutiquement et/ou dermatologiquement acceptable est choisi dans le groupe comprenant un agent émulsifiant, un polymère et une combinaison de ceux-ci,
l'agent émulsifiant est choisi dans le groupe comprenant : un agent tensioactif cationique, un agent tensioactif anionique, un agent tensioactif zwitterionique, un agent tensioactif amphotère, le polyoxyl 20 cetostearyl ether, et leurs combinaisons, et
le polymère est choisi dans le groupe comprenant : un carbomère, le polyéthylène glycol, un polymère d'acrylate, un polymère de méthacrylate, la polyvinylpyrrolidone, un copolymère à base de méthacrylate de butyle et de méthacrylate de méthyle, l'acétate de vinyle, l'acétate de polyvinyle, la cellulose, la gomme, l'alginate, le phtalate d'acétate de cellulose, le butyrate d'acétate de cellulose, le phtalate d'hydroxypropylméthylcellulose, et leurs combinaisons.

6. Composition selon la revendication 1,
comprenant en outre un ou plusieurs excipients choisis dans le groupe comprenant : un antioxydant, un conservateur, un humectant et un plastifiant.

7. Composition selon la revendication 1,
la composition étant sensiblement non irritante pour la peau du sujet.

8. Composition selon la revendication 1,
la composition étant stable au moins pendant la période d'environ 6 mois à 40° C ou au moins pendant une période de 24 mois à 25° C.

9. Composition selon la revendication 1, dans laquelle
le composé de bétaméthasone est le dipropionate de bétaméthasone.

10. Composition selon la revendication 1, dans laquelle
l'impureté aldéhyde énol dans la composition est inférieure à environ 1% pendant une période d'au moins 12 mois à 25° C,
l'impureté aldéhyde énol dans la composition est inférieure à environ 1% pendant une période d'au moins 18 mois à 25° C,
l'impureté aldéhyde énol dans la composition est inférieure à environ 1% pendant une période d'au moins 24 mois à 25° C.

11. Composition selon la revendication 1,
la composition assurant la rétention de bétaméthasones dans les couches de la peau de 0,1 % à 10 % de la dose appliquée.

12. Composition selon la revendication 1,
la composition étant une composition de dépôt cutané.

13. Composition selon la revendication 1,
la composition étant une émulsion choisie dans le groupe comprenant :
une émulsion à base aqueuse et une émulsion d'huile dans l'eau.

14. Composition de pulvérisation topique destinée utilisée pour le traitement de la dermatite atopique, de la dermatite séborrhéique, de l'eczéma et du psoriasis,
la composition de pulvérisation topique étant administrée par les étapes suivantes consistant à :
utiliser un dispositif ayant la composition de pulvérisation topique de la revendication 1, et
administrer une pulvérisation de cette composition directement sur une partie affectée de la peau d'un sujet qui en a besoin.

15. Composition de pulvérisation topique utilisée selon la revendication 14, dans laquelle
le procédé a des caractéristiques de pulvérisation d'une forme de pulvérisation à cône plein à grand angle, ayant un premier axe de 35 mm à 60 mm, et un second axe de 35 mm à 55 mm, le rapport entre le premier et le second axe étant de 1 à 1,5.

16. Composition de pulvérisation topique utilisée selon la revendication 15,
le procédé distribuant de 65 mg à 210 mg de composition par action,
la composition de pulvérisation permettant environ 230 à environ 270 d'actions jusqu'à l'épuisement de la composition dans le dispositif.

17. Composition de pulvérisation topique utilisée selon la revendication 14,
la distance d'administration étant de 20 mm à 60 mm entre la peau du sujet et le dispositif, l'angle de pulvérisation étant de 50 à 70 degrés par rapport à la peau du sujet.

18. Composition de pulvérisation topique utilisée selon la revendication 14,
la composition formant un dépôt sur la peau sans former de film occlusif, ou la composition est une émulsion à base aqueuse.

19. Composition de pulvérisation topique utilisée selon la revendication 14,
le psoriasis étant un psoriasis en plaques, modéré à sévère.
